# EUROPEAN PATENT APPLICATION

(11) **EP 1 020 805 A2**
(43) Date of publication of application: **19.07.2000**
(21) Application number: 00300011.4
(22) Date of filing: 05.01.2000
(51) Int. Cl.: G06F 17/60

(54) **Coding system and method for obtaining electronic online information related to coded physical items**

(30) Priority: 11.01.1999 US 228439
(71) Applicant: International Business Machines Corporation, Armonk, NY 10504 (US)
(72) Inventor: Chandrasekhar, Narayanaswami c/o IBM U.K. Ltd., Winchester, Hampshire SO21 2JN (GB)
(74) Representative: Moss, Robert Douglas

(57) **Abstract**

A method for providing a link between physical items with online computer information related to the physical items by assigning coded data to each physical item. The coded data may be printed somewhere appropriate on the physical item. The code uniquely associates the physical items with stored computer data related to the physical item that is accessed via the internet. When the code is entered into a computer by a user, the electronic data related to the coded physical item is retrieved by accessing it over the internet or similar communication network and the electronic data is then stored in the user's electronic archive.

## Description

### TECHNICAL FIELD OF THE INVENTION

The present invention relates to the coding of physical items and more particularly, to a coding technique for connecting a physical item and an online computer electronic information related to the physical item.

### BACKGROUND OF THE INVENTION

Coded indicia such as bar codes and the like have been used with physical items such as retail goods, articles of manufacture and printed documents for many purposes such as classification, inventory, selling price, authorship, mailing and the like. The present invention provides a novel coding technique for a physical item that links the physical item with online electronic computer information related to the physical. The electronic information may then be displayed, printed or similarly communicated to a user.

### DISCLOSURE OF THE INVENTION

An object of the present invention is to provide a method for providing a link between physical items with an online computer online information related to the physical items by assigning a code to each physical item. The code is affixed somewhere appropriate on the physical item.

The code uniquely associates the physical items with stored computer data related to the physical item that is accessed via the internet. When the code is entered into a computer by a user, the electronic data related to the coded physical item is retrieved by accessing it over the internet or similar communication network and the electronic data is then stored in the user's electronic archive.

### BRIEF DESCRIPTION OF THE DRAWINGS

The foregoing aspects and other features of the present invention are explained in the following description, taken in connection with the accompanying drawings, wherein:

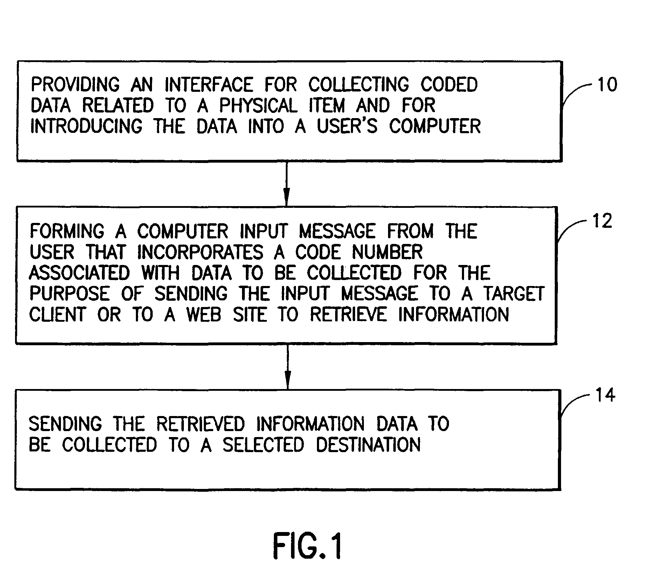

Fig. 1 is a flow chart illustrating the steps of a method according to the principles of the present invention.

### DETAILED DESCRIPTION OF THE INVENTION

With the advent of the internet and the world wide web, a large amount of information can be made available to a computer user relative to physical items. For example, if the physical item is a particular wristwatch, a code may be associated with the physical item indicating the manufacturer and model. In the present invention a computer user would enter the code into the Internet via the computer keyboard or a scanner to access Internet sites associated with the wristwatch that could provide the user with information such as the present value of the watch, where and how to purchase such watch, where the watch can be sent for repairs, a parts list for the wristwatch, and a great deal more. Likewise, publishers of many newspapers and magazines have provided both printed and electronic online versions of their media. Printed versions are clearly easier to read due to the higher quality of printed paper than a computer display. However, an electronic version is far superior for searching and for archival.

Relative with respect to the embodiment of the invention to documents, the present invention provides method that is employed where a user reads an article in a printed format and wants to preserve the document. However, the user either does not have the physical space to save the printed article or would like to avoid clutter and would prefer a copy to be stored on a computer in digital form to allow the user access and search the document later.

The following description is directed to one embodiment of the present invention wherein the physical item is a document and wherein the invention provides a method that reduces the need to collect and store paper documents and automates the process of document clipping. It also makes it easier to search documents. The online version and the printed version need not correspond exactly. The online version may augment the printed version with more data, related data, follow-up articles, multimedia presentations, and similar things that cannot be captured on print, etc.

Referring to Fig. 1, an illustration of a flow chart is shown disclosing an embodiment of the steps of the method recording to the present invention. In step 10 of Fig. 1, an interface is provided for collecting coded data related to a physical item and for also introducing the data into a user's computer. Then, in step 12, a computer input message from the user is formed that incorporates a code number associated with data to be collected for the purpose of pending the input message to a target client or to a web site to retrieve information. In step 14, the retrieved information is then sent to a selected destination.

A specific example of the present invention, the information to be retrieved may be a document desired by the user. The input from the user incorporates a code number for the physical item (document) desired by the user. The provider system reads the code number, retrieves an electronic copy of the desired document and displays it on the user's monitor. The document code number may be a simple number or bar code. When it is a number the user may enter it manually via the computer keyboard and when it is a bar code, a scanner can scan the number and thus the user does not need to remember the code number, When the document code number is inputted, by a scanner connected to the user's computer, it may initiate a connection to the online data automatically to retrieve the online document. It may also function to store the retrieved on line information in the appropriate location in the user's electronic archive. A combination of both manual and scanner inputs can be employed if desired. For example, a plurality of document code numbers may be scanned at one time. Then when the user connects the scanner to the computer, all the captured document codes will be processed one by one.

More particularly, the user input may also include a target client as well as the document code in the manual or scanned input. A target client is the client to which to send the document code. For example, the target client may be at the machine to which the scanner is directly interfaced to or the target client may be a server on the internet or intranet computer to which the user is connected to. The target client may be identified by a uniquely assigned identification address such as its IP address. When the target client receives an input with a client identification combined with a document code, the target client accesses a stored table that maps a document to URLs in order to identify a corresponding URL. The target client makes a web (http, shttp, ftp, etc.) request for the corresponding URL and instructs that the URL data be sent either directly to the user's computer, or via the target client to the user's computer.

A bar code for use with the present invention may be composed, for example, of a left half and a right half of code. The left half has a quiet zone (nine white modules), a guard pattern (3 modules), category (seven modules), and manufacturer (35 modules) the right half has product identification (35 modules), check digit (7 modules), guard pattern (3 modules), and a quiet zone (9 modules). The left and right half are separated by a centre guard pattern (5 modules).

The method of the present invention completely automates the process of connecting printed material with corresponding online electronic versions of the material and reduces time and effort for the user.

It should be noted that some printed articles provide web page addresses next to the printed version of the document. The method of the present invention also completely automates this process completely and reduces the burden for the user.

As previously discussed, the present invention can be extended for use with other physical items. In addition, the present invention can be employed with other technologies. For example, the disclosed method can be extended to radio excerpts as well. Imagine that you are listening to a radio station, such as when travelling in an automobile, and you listen to something important. For example, you hear that tickets are on sale for a concert you would like to go to. A feature of the present invention is that you can just hit the record button on your radio. The radio records the information about the concert, such as on a smart card, and stores enough data to be able to provide an online version of ticket sales counter. When you reach home or the nearest store where you can buy your ticket, you unplug the radio or a portion of the radio such as the smart card where data is recorded from your car and plug it in to either your computer or your phone line and you will be taken to the online store where you can buy your ticket. So the whole process of buying your ticket is dramatically simplified. No need to note down the phone number or the web address when you are driving. This concept can be applied to other things as well for example to news items, sports scores, stock reports, etc. In each case, web/Internet data will be transmitted along with the radio signal which can be recorded by the radio. The radio itself may not be connected to the Internet. When the user hits the record button, the radio records the associated Internet data. The Internet data that has been recorded can be used to connect to the internet at a later point in time, as explained in the above example, to get the user more information from the Internet.

The radio itself or part of it is made detachable and has an interface to connect to a computer via say a serial, parallel, USB or other part; or a phone. If connected to a computer or a screen phone, the user will be taken to the corresponding web site. If connected to a phone it may take you to a telephone agent for the company.

The Internet data itself could include a web address for the associated piece of information or it could be a number or a bar code.

Alternatively, the Internet data could be constructed from auxiliary data such as the frequency of the radio station, the local time of day, the latitude and longitude of the place where the information was recorded. This information can then be transmitted to a central server. An Internet search is performed on the server with the transmitted data and the online information is located. The search could be a simple search if the service provider and the broadcaster has a standardized method of representing the Internet data. The search could be more complex if no agreed to standard exists, but then the broadcaster has to make no changes to his broadcast. Other items such as physical objects may have id tags as well that can be read by a tag reader and transmitted to a device capable of Internet access. For example, a child's toy could get an update from a web site on the Internet, say the vocabulary of a talking doll could be changed from English to German.

## Claims

1. A method for obtaining online computer electronic information related to coded physical items comprising the steps of:
collecting coded data related to a physical item and introducing the data into a user's computer;
sending a message from the user's computer that incorporates a code number associated with the data collected to a target client or directly to a web site to retrieve information, based on the data collected,
sending the retrieved information to a selected destination.

2. A method according to claim 1 including the step of forming the information to be retrieved based on the data collected.

3. A method according to claim 1 or claim 2 wherein the step of sending the retrieved information to a selected destination includes sending the information to the computer user and displaying the retrieved information on the computer user's display screen.

4. A method according to any preceding claim wherein the code number associated with a physical item is a bar code disposed on the physical item.

5. A method as claimed in any preceding claim wherein the message from the user's computer includes an address for a specific target provider system.

6. A method as claimed in any preceding claim wherein the message from the user's computer is a radio signal transmitted by a computer input device.

7. A method according to claim 6 wherein the radio signal comprises radio excerpts of internet data recorded on a detachable memory device and containing information related to the radio broadcast.

8. A method according to claim 7 wherein the radio signal may be associated with a video transmission signal.

9. A method according to any preceding claim wherein the retrieved information is classified or categorized by item number, date retrieved, size and the like and stored in the user's computer.

10. A computer program for linking physical items and corresponding computer online information to the physical items, comprising:
computer program code for causing a computer to collect coded data related to a physical item and for introducing the data into the computer;
computer program code for causing a computer to form a computer input message that incorporates a code number associated with data to be collected for the purpose of sending the input message to a target client or to a web site to retrieve information; and
computer program code for sending the retrieved information data to be collected to a selected destination.
